# EUROPEAN PATENT APPLICATION

(11) **EP 2 644 608 A1**
(43) Date of publication of application: **02.10.2013**
(21) Application number: 13160798.8
(22) Date of filing: 25.03.2013
(51) Int. Cl.: C07D 413/12

(54) **One-pot process for the synthesis of iloperidone**

(30) Priority: 29.03.2012 IT MI20120511
(71) Applicant: Procos S.p.A., 28062 Cameri NO (IT)
(72) Inventor: Bettoni, Piergiorgio, 15033 CASALE MONFERRATO (AL) (IT); Roletto, Jacopo, 10135 TORINO (IT); Paissoni, Paolo, 10040 DRUENTO (TO) (IT)
(74) Representative: Minoja, Fabrizio

(57) **Abstract**

Disclosed is a process for the synthesis of iloperidone starting from 4-hydroxy-3-methoxy acetophenone (acetovanillone), 1-chloro-3-bromo propane and 6-fluoro-3-(4-piperidinyl)-1,2-benzisoxazole hydrochloride, using a one-pot method.

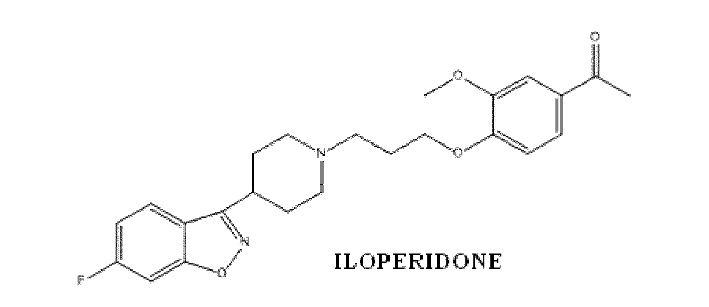

Said process is performed without any intermediate isolation, and is particularly advantageous from the environmental standpoint and in terms of yields, productivity and the purity of the product obtained, both in the reaction mixture and in the crystal isolated.

## Description

### SUMMARY OF THE INVENTION

The present invention relates to an advantageous process for the preparation of iloperidone, wherein the intermediate 1-[4-(3-chloropropoxy)-3-methoxyphenyl]-ethanone is not isolated but used in the next step with no need to conduct any work-up, isolation or purification step.

### BACKGROUND TO THE INVENTION

Iloperidone (1-[4-[3-[4-(6-fluoro-1,2-benzisoxazole-3-yl)-1-piperidinyl]propoxy]-3-methoxyphenyl]ethanone) is an atypical new-generation antipsychotic medicament belonging to the class of piperidinyl-benzisoxazole derivatives, which is used to treat schizophrenia, bipolar disorder and other psychiatric conditions. Iloperidone acts as a serotonin/dopamine receptor antagonist (5-HT_{2A}/D₂).

The synthesis of iloperidone is described in USRE39198 (corresponding to EP 0 402 644 example 3) according to the following synthesis scheme:

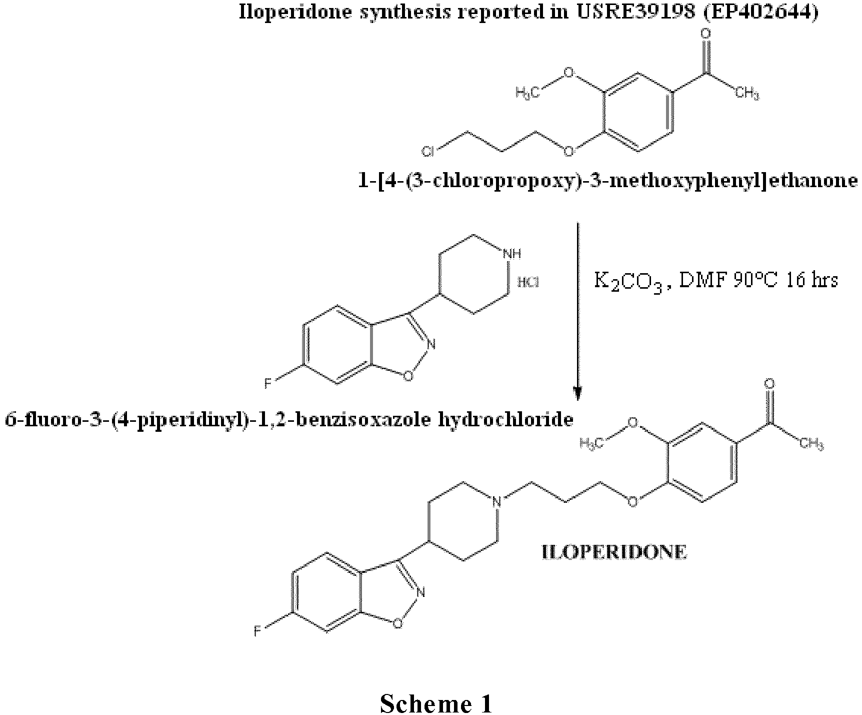

In agreement with said patent, the intermediate isolated, 1-[4-(3-chloropropoxy)-3-methoxyphenyl]ethanone, is reacted with 6-fluoro-3-(4-piperidinyl)-1,2-benzisoxazole hydrochloride in N,N-dimethyl formamide at 90°C for 16 hours. When the reaction is complete, the mixture is poured into water and extracted with ethyl acetate. The crude product thus obtained is crystallised twice from ethanol to give crystallised iloperidone with a total yield of 58%.

The yield of this process is very low; moreover, the process begins with two isolated intermediates, and requires an aqueous extractive work-up step with an increase in volumes and consequent reduction in the productivity and efficiency of the process. Said process also requires a double crystallisation step to obtain a beige product. The quality levels obtained are not described in the text of the example, but a beige color does not suggest a high-quality product, as iloperidone is a white substance.

The synthesis of intermediate 1-[4-(3-chloropropoxy)-3-methoxyphenyl]ethanone is disclosed in US4366162. Example 1 describes the preparation of said intermediate by reacting acetovanillone with 1-bromo-3-chloropropane in acetone with potassium carbonate. At the end of the reaction the resulting product is purified by distillation and obtained as an oily intermediate which is left to stand in order to obtain the solid intermediate.

The synthesis of intermediate 1-[4-(3-chloropropoxy)-3-methoxyphenyl]ethanone is also disclosed in US4810713. Preparation 12 describes the synthesis of said intermediate from acetovanillone and 1-bromo-3-chloropropane in sodium hydroxide alkalinized water. At the end of the reaction the product obtained is extracted in toluene, the organic phases are washed with basic aqueous solutions and finally, the intermediate 1-[4-(3-chloropropoxy)-3-methoxyphenyl]ethanone is crystallised with the aid of diisopropyl ether. The intermediate isolated is then recrystallised twice from cyclohexane and twice from petroleum ether.

An alternative process for the synthesis of iloperidone is reported in CN 102070626.

Scheme 2 shows the synthesis procedure:

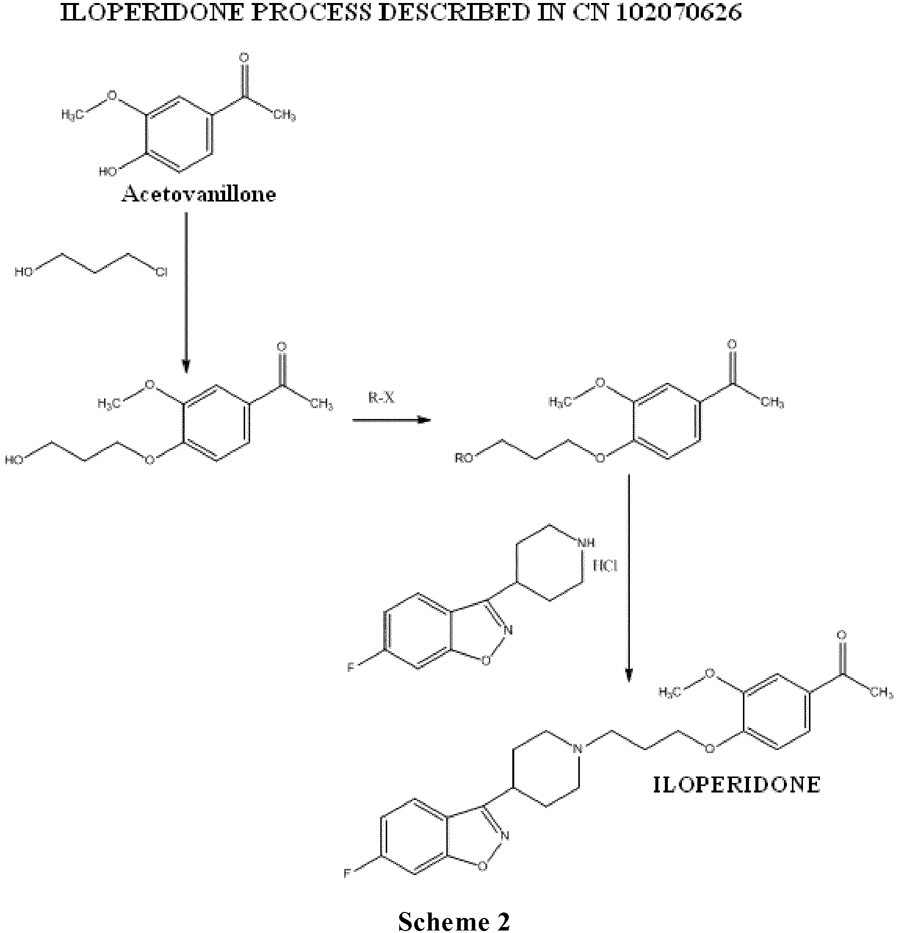

The decision to alkylate acetovanillone with 1-chloro-3-propanol requires an extra synthesis step (to convert the OH group to an OR leaving group) compared with the procedure reported by the combination of patents USRE39198 (EP402644) and US4366162 / US4810713, making said process less efficient from the economic standpoint.

WO201106175 discloses an alternative iloperidone synthesis process as reported in Scheme 3:

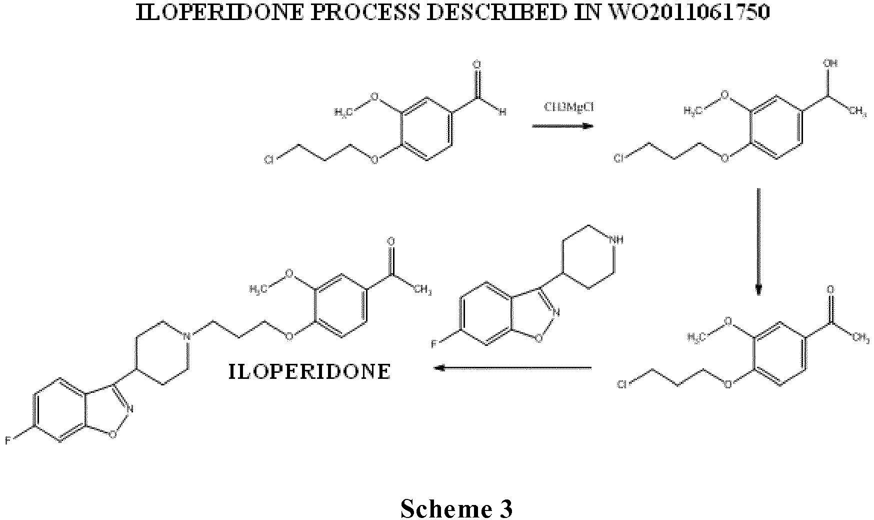

Said process uses reagents such as methyl magnesium chloride to effect the Grignard reaction to convert the aldehyde group to a secondary alcohol group, which are much more complicated to manage on an industrial scale than the synthesis methods previously described. Moreover, the oxidation reaction of the next step uses reagents such as chromic acid or potassium permanganate, which have a very high environmental impact and very low industrial applicability.

WO2011055188 discloses a process for the synthesis of iloperidone comparable to the one reported in USRE39198 from two isolated intermediates 1-[4-(3-chloropropoxy)-3-methoxyphenyl]ethanone and 6-fluoro-3-(4-piperidinyl)-1,2-benzisoxazole hydrochloride. The same patent application also gives preparation examples of the intermediate 1-[4-(3-chloropropoxy)-3-methoxyphenyl]ethanone isolated as crystalline solid by procedures similar to those known in the literature.

CN 101824030 reports an iloperidone synthesis method similar to that of CN 102070626 which involves the same problems of inefficiency due to the additional step of inserting the leaving group required for alkylation with 6-fluoro-3-(4-piperidinyl)-1,2-benzisoxazole hydrochloride.

CN 101781243 discloses an alternative iloperidone synthesis process as reported in Scheme 4.

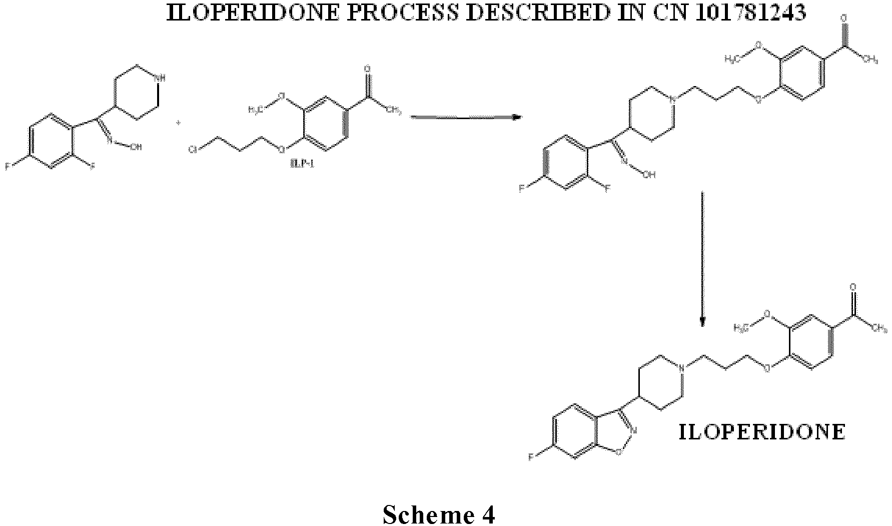

Said process is not advantageous compared with the preceding processes as the intermediate with the oxime group, due to the nature of this functional group, is particularly liable to degradation due to the action of numerous factors such as the presence of metals, acid pHs and basic pHs.

CN101768154 discloses a process for the synthesis of iloperidone comparable to the one reported in USRE39198 from two isolated intermediates, 1-[4-(3-chloropropoxy)-3-methoxyphenyl]ethanone and 6-fluoro-3-(4-piperidinyl)-1,2-benzisoxazole hydrochloride.

CN 101735208 describes a process for the synthesis of iloperidone comparable to the one reported in CN 101781243, namely through the intermediate with the functional oxime group.

IN 2007MU01980 discloses a process for the synthesis of iloperidone comparable to the one reported in USRE39198 from two isolated intermediates, 1-[4-(3-chloropropoxy)-3-methoxyphenyl]ethanone and 6-fluoro-3-(4-piperidinyl)-1,2-benzisoxazole hydrochloride.

WO 2010031497 describes an alternative iloperidone synthesis process as reported in Scheme 5.

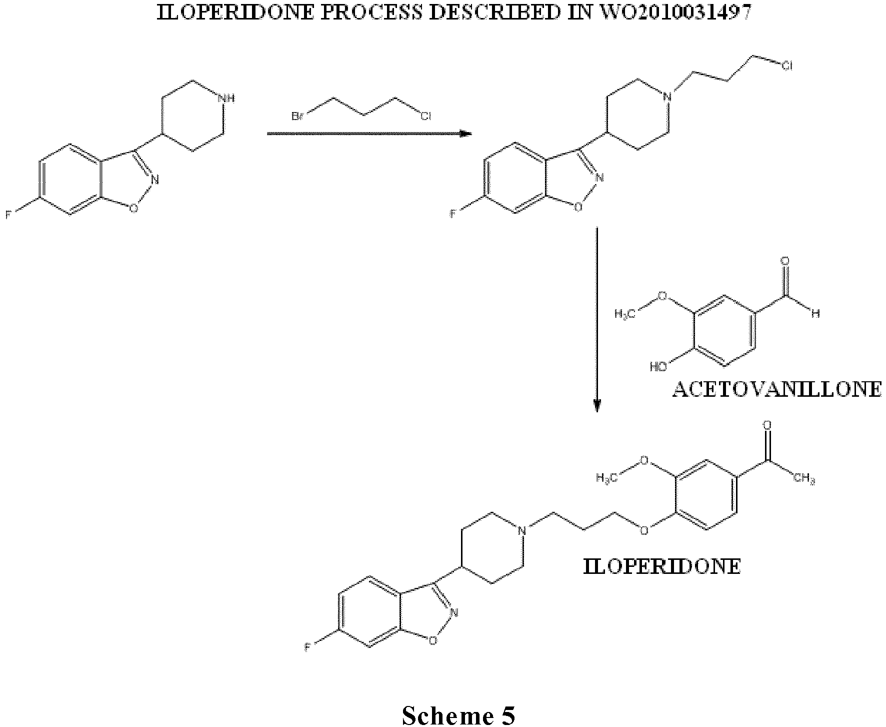

The considerable economic disadvantage of the process reported in WO2010031497 is based on the fact that by reversing the order of alkylation and performing that of intermediate 6-fluoro-3-(4-piperidinyl)-1,2-benzisoxazole hydrochloride first, a greater loss of yield is generated on this intermediate which, according to the literature, is more difficult to synthesise and consequently more expensive than the intermediate 1-[4-(3-chloropropoxy)-3-methoxyphenyl]ethanone, with a globally greater economic inefficiency of the iloperidone preparation process.

CN 102212063 discloses a process for the synthesis of iloperidone with the same arrangement of the synthesis steps as patent application WO 2010031497.

WO2011154860 describes a process for the synthesis of iloperidone wherein a phase transfer catalyst is used to prepare the intermediate 1-[4-(3-chloropropoxy)-3-methoxyphenyl]ethanone which, as in all the other preparation examples previously described, is crystallised, isolated and dried before use in the next step with 6-fluoro-3-(4-piperidinyl)-1,2-benzisoxazole hydrochloride. Scheme 6 shows the synthesis scheme of the process of WO2011154860.

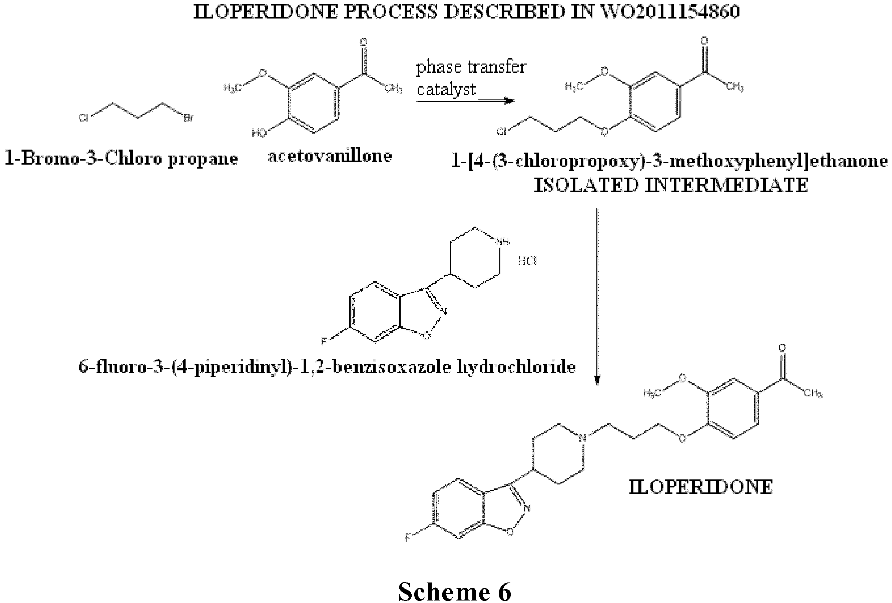

On the basis of the information collected from the literature, there is an evident need to find a process for the synthesis of iloperidone with high purity which offers greater economic efficiency for implementation on an industrial scale; by "greater efficiency" is meant not only an increase in molar yield but also a reduction in the number of unit operations such as filtrations and isolations of process intermediates, and in particular of the intermediate 1-[4-(3-chloropropoxy)-3-methoxyphenyl]ethanone.

### DESCRIPTION OF THE INVENTION

In the synthesis of 1-[4-(3-chloropropoxy)-3-methoxyphenyl]ethanone by alkylation of acetovanillone with 1-bromo-3-chloropropane, purification of said intermediate is mandatory when a significant molar excess of 1-bromo-3-chloropropane is used with respect to acetovanillone, as it is the case of the synthesis described in Example 1 of US4366162, wherein 1.32 molar equivalents of the latter reagent are used. This as in the subsequent step the excess of 1-bromo-3-chloropropane would react with 6-fluoro-3-(4-piperidinyl)-1,2-benzisoxazole hydrochloride to give an iloperidone contaminated by impurities difficult to remove.

An advantageous process for the preparation of iloperidone has now been found wherein using a stoichiometric ratio between acetovanillone and 1-bromo-3-chloropropane close to 1.0 the intermediate 1-[4-(3-chloropropoxy)-3-methoxyphenyl]-ethanone is surprisingly not isolated but used in the next step with no need to conduct any work-up, isolation or purification step. The process according to the invention is exemplified in Scheme 7.

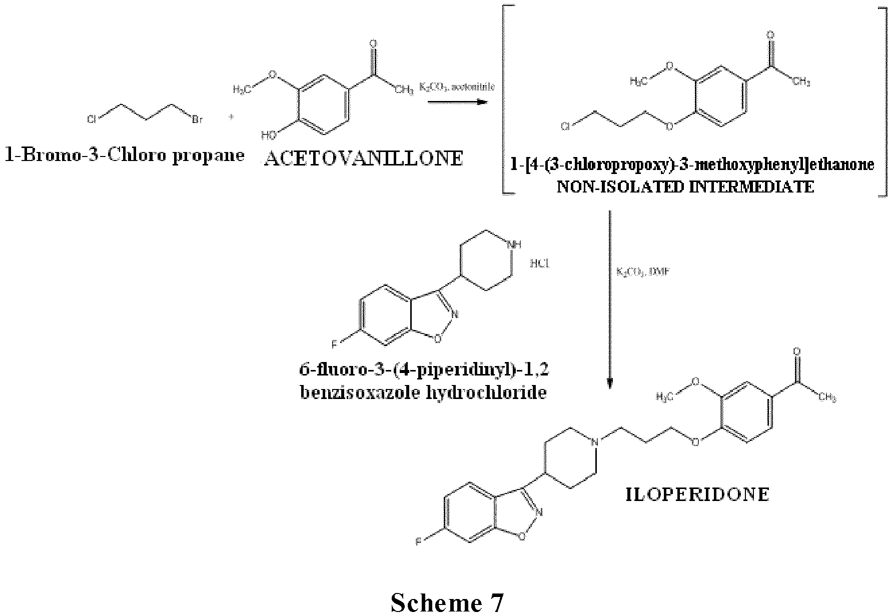

The object of the present invention is a process for the preparation of iloperidone comprising the following steps:
i. alkylation of acetovanillone with 1-bromo-3-chloropropane to give the intermediate 1-[4-(3-chloropropoxy)-3-methoxyphenyl]ethanone in the presence of an inorganic base;
ii. alkylation of 6-fluoro-3-(4-piperidinyl)-1,2-benzisoxazole hydrochloride by the intermediate 1-[4-(3-chloropropoxy)-3-methoxyphenyl]-ethanone obtained in the first step, in the presence of an inorganic base, to give a reaction mixture containing iloperidone;
iii. isolation of iloperidone from the reaction mixture obtained in the second step;
**characterised in that** in the first step 0.9-1.2 molar equivalents, preferably 1.0-1.16 molar equivalents, even more preferably 1.0 equivalents of 1-bromo-3-chloropropane with respect to acetovanillone are used;
and further **characterised in that** the intermediate 1-[4-(3-chloropropoxy)-3-methoxyphenyl]ethanone obtained in the first step is not isolated.

In a preferred embodiment of the invention, iloperidone is isolated from the reaction mixture obtained in the second step simply by filtering the reaction mixture to remove inorganic salts, followed by concentration of the reaction mixture, resulting in the separation of iloperidone with high yields and purity.

The first synthesis step, wherein the non-isolated intermediate 1-[4-(3-chloropropoxy)-3-methoxyphenyl]ethanone is produced, takes place in an organic solvent or mixtures of solvents selected from acetonitrile, N,N-dimethylformamide, N,N-dimethylacetamide, N-methylpyrrolidone, preferably acetonitrile or mixtures of acetonitrile with N,N-dimethylformamide in the presence of an inorganic base, preferably potassium carbonate, potassium bicarbonate, sodium carbonate and sodium bicarbonate.

Preferably acetovanillone is added to a previously prepared solution of 1-bromo-3-chloropropane in acetonitrile or in a mixture of acetonitrile with N,N-dimethylformamide in the presence of an inorganic base.

The use in the first step of the present process of a stoichiometric ratio close to 1.0 as above defined between 1-bromo-3-chloropropane and acetovanillone, the reversal of the order of addition of acetovanillone and 1-bromo-3-chloropropane to the reaction mixture and the replacement of acetone with acetonitrile or acetonitrile/N,N,-dimethylformamide mixtures result in a significant improvement of the conversion of acetovanillone to 1-[4-(3-chloropropoxy)-3-methoxyphenyl]ethanone which can be converted in an one-pot process to an iloperidone characterized by an excellent purity profile.

The second synthesis step, wherein iloperidone is produced by alkylation of 6-fluoro-3-(4-piperidinyl)-1,2-benzisoxazole hydrochloride by the non-isolated intermediate 1-[4-(3-chloropropoxy)-3-methoxyphenyl]ethanone, takes place in an organic solvent or a mixture of solvents selected from acetonitrile, N,N-dimethylformamide, N,N-dimethylacetamide and N-methylpyrrolidone, preferably acetonitrile or mixtures of acetonitrile with N,N-dimethylformamide in the presence of an inorganic base, preferably potassium carbonate, potassium bicarbonate, sodium carbonate and sodium bicarbonate.

Typically, the process according to the invention involves synthesising iloperidone by alkylation of 6-fluoro-3-(4-piperidinyl)-1,2-benzisoxazole hydrochloride by the intermediate 1-[4-(3-chloropropoxy)-3-methoxyphenyl]ethanone directly from the reaction mixture originating from the first synthesis step, without any work-up, isolation or purification step, wherein an organic solvent selected from acetonitrile, N,N-dimethylformamide, N,N-dimethylacetamide, N-methylpyrrolidone, an inorganic base, preferably potassium carbonate, potassium bicarbonate, sodium carbonate or sodium bicarbonate, and the raw material 6-fluoro-3-(4-piperidinyl)-1,2-benzisoxazole hydrochloride, are added to the reaction mixture of the first synthesis step.

The order of addition of the organic solvent, the inorganic base and the raw material 6-fluoro-3-(4-piperidinyl)-1,2-benzisoxazole hydrochloride can also be different from that reported above.

The iloperidone obtained is isolated directly from the reaction mixture of the second synthesis step by simple filtration of the reaction mixture to remove the inorganic salts, followed by concentration of the reaction mixture by evaporation of the solvent, resulting in the separation of iloperidone. The quality of the product obtained exceeds 99%.

According to a preferred embodiment of the invention, the process is performed as follows:
Typically, 1 molar equivalent of acetovanillone is added to an organic solution of 0.9-1.2 molar equivalents of 1-bromo-3-chloropropane, preferably 1.0-16molar equivalents, even more preferably 1.0 equivalents, in the presence of an inorganic base, preferably potassium carbonate, in the amount of 0.9-2.0 molar equivalents, preferably 1.0-1.2 molar equivalents. The reaction is conducted in organic solvent or mixtures of solvents selected from acetonitrile, N,N-dimethylformamide, N,N-dimethylacetamide, N-methylpyrrolidone, preferably acetonitrile or a mixture of acetonitrile and N,N-dimethylformamide in the temperature range between 45°C and the reflux temperature of the reaction mixture, preferably at the temperature of 75°-85°C. 5-25 Volumes of solvent are used, preferably 10-20 volumes with respect to the amount of acetovanillone. The reaction is controlled by conventional analysis techniques, such as UPLC analysis using an ACQUITY BEH C18 column and water/acetonitrile/0.1% trifluoroacetic acid as eluent phase. After completion of the reaction, an inorganic base, preferably potassium carbonate, in an amount between 1.0 and 4.0 molar equivalents with respect to 1 molar equivalent of starting acetovanillone, preferably between 2.0 and 3.0 molar equivalents, is added to the reaction mixture containing the intermediate 1-[4-(3-chloropropoxy)-3-methoxyphenyl]ethanone. 6-Fluoro-3-(4-piperidinyl)-1,2-benzisoxazole hydrochloride is added to the reaction mixture in an amount between 0.85 and 1.20 molar equivalents with respect to 1 molar equivalent of starting acetovanillone, preferably between 0.90 and 1.00 molar equivalents. The resulting mixture is diluted with an organic solvent selected from acetonitrile, N,N-dimethylformamide, N,N-dimethylacetamide and N-methylpyrrolidone and heated to a temperature range between 70°C and the reflux temperature of the reaction mixture, preferably to the temperature of 80- 95°C. 1- 10 Volumes of solvent are used, preferably 3- 5 volumes with respect to the starting amount of acetovanillone. The reaction is controlled by conventional analysis techniques, such as UPLC analysis using an ACQUITY BEH C18 column and water/acetonitrile/0.1% trifluoroacetic acid as eluent phase. After completion of the reaction, the reaction mixture containing the iloperidone is cooled to the temperature of 40-60°C, preferably 45-55°C, and filtered; the solid filtrate is discarded as it consists mainly of inorganic salts. The clear filtered solution is concentrated under vacuum to a small volume and cooled to the temperature of 0-10°C. The resulting suspension is filtered to isolate the iloperidone. The solid filtrate is dried under vacuum at the temperature of 30-90°C, preferably 55-60°C, to obtain iloperidone with a purity exceeding 99%. The resulting solid can be further purified if necessary by recrystallisation from ethanol or other solvents known in the literature.

The process according to the invention is particularly advantageous in that, unlike the processes described in the literature, it is carried out without isolating the intermediate 1-[4-(3-chloropropoxy)-3-methoxyphenyl]ethanone, and also allows the product iloperidone to be obtained in high yields and a purity exceeding 99% directly from the reaction mixture by simple filtration of the reaction mixture followed by concentration thereof and filtration of the product, thus considerably simplifying the work-up steps described in the literature.

The invention is illustrated in detail in the following examples.

### Example 1: synthesis of iloperidone

Acetovanillone (45 g, 0.27 mol) is added to a suspension of potassium carbonate (50 g, 0.36 mol), acetonitrile (860 ml) and 1-bromo-3-chloropropane (42.6 g, 0.27 mol). The mixture is heated at the temperature of 75-80°C and monitored by UPLC. After completion of the reaction, potassium carbonate (70 g, 0.50 mol), 6-fluoro-3-(4-piperidinyl)-1,2-benzisoxazole hydrochloride (72 g, 0.28 mol) and 250 ml of N,N-dimethylformamide are added. The mixture is heated at the temperature of 90-95°C and monitored by UPLC. After completion of the reaction, the resulting mixture is cooled to 55°C and the inorganic salts are filtered off. The clear solution obtained is concentrated under vacuum to a weight of 321 g, and cooled to 5°C. The resulting suspension is filtered. The solid isolated is dried under low pressure at 55°C to obtain iloperidone (85 g, 0.20 mol) as a white solid with a purity exceeding 99%. Molar yield from acetovanillone to iloperidone: 74%.
UPLC-MS [M+H+] = 427
1H-NMR (in DMSO) (chemical shifts expressed in ppm with respect to the TMS signal): 2.06-1.78 (6H, m); 2.13 (2H, m); 2.49 (2H, t); 2.52 (2H, m); 2.97 (2H, m); 3.11 (1H, tt); 3.83 (3H, s); 4.12 (2H, t); 7.06 (1H, d); 7.22 (1H, m); 7.46 (1H, d); 7.61-7.58 (2H, m); 7.94 (1H, dd).

### Example 2: synthesis of iloperidone

Acetovanillone (50 g, 0.30 mol) is added to a suspension of potassium carbonate (60 g, 0.43 mol), acetonitrile (800 ml), N,N-dimethylformamide (200 ml) and 1-bromo-3-chloropropane (55.2 g, 0.35 mol). The mixture is heated at the temperature of 75-80°C and monitored by UPLC. After completion of the reaction, potassium carbonate (80 g, 0.58 mol), 6-fluoro-3-(4-piperidinyl)-1,2-benzisoxazole hydrochloride (90 g, 0.35 mol) and 100 ml of N,N-dimethylformamide are added. The mixture is heated at the temperature of 90-95°C and monitored by UPLC. After completion of the reaction, the resulting mixture is cooled to 55°C and the inorganic salts are filtered off. The clear solution obtained is concentrated under vacuum to a weight of 370 g, and cooled to 5°C. The resulting suspension is filtered. The solid isolated is dried under low pressure at 55°C to obtain iloperidone (97 g, 0.23 mol) as a white solid with a purity exceeding 99%. Molar yield from acetovanillone to iloperidone: 76%.

## Claims

1. A process for the preparation of iloperidone comprising the following steps:
i. alkylation of acetovanillone with 1-bromo-3-chloropropane to give intermediate 1-[4-(3-chloropropoxy)-3-methoxyphenyl]ethanone in the presence of an inorganic base;
ii. alkylation of 6-fluoro-3-(4-piperidinyl)-1,2-benzisoxazole hydrochloride by intermediate 1-[4-(3-chloropropoxy)-3-methoxyphenyl]ethanone obtained in the first step, in the presence of an inorganic base, to give a reaction mixture containing iloperidone;
iii. isolation of iloperidone from the reaction mixture obtained in the second step;
**characterised in that** in the first step 0.9-1.2 molar equivalents, preferably 1.0-1.16 molar equivalents, even more preferably 1.0 equivalents of 1-bromo-3-chloropropane with respect to acetovanillone are used;
and further **characterized in that** the intermediate 1-[4-(3-chloropropoxy)-3-methoxyphenyl]ethanone is not isolated.

2. Process according to claim 1 wherein the isolation of iloperidone comprises filtration of the reaction mixture to remove inorganic salts followed by concentration of the reaction mixture.

3. Process according to claim 1, wherein the first and second steps are carried out in an organic solvent or in a mixture of organic solvents independently selected from acetonitrile, N,N-dimethylformamide, N,N-dimethylacetamide, N-methylpyrrolidone, preferably acetonitrile or mixtures of acetonitrile with N,N-dimethylformamide.

4. Process according to claim 1 wherein the inorganic base of the first and second steps is independently selected from potassium carbonate, potassium bicarbonate, sodium carbonate and sodium bicarbonate, preferably potassium carbonate.

5. Process according to claims 1-4 wherein in the first step 1 molar equivalent of acetovanillone is added to an organic solution of 0.9 -1.2 molar equivalents, preferably 1.0-1.16 molar equivalents, even more preferably 1.0 equivalents of 1-bromo-3-chloropropane of 1-bromo-3-chloropropane, in the presence of an inorganic base in an amount ranging from 0.9 - 2.0 molar equivalents, at a temperature ranging from 45°C to the reflux temperature of the reaction mixture.

6. Process according to claims 1-5 wherein in the second step the inorganic base is used in an amount ranging from 1.0 - 4.0 molar equivalents with respect to 1 molar equivalent of starting acetovanillone and 6-fluoro-3-(4-piperidinyl)-1,2-benzisoxazole hydrochloride is used in an amount ranging from 0.85-1.20 molar equivalents with respect to 1 molar equivalent of starting acetovanillone, at a temperature ranging from 70°C to the reflux temperature of the reaction mixture.
